# EUROPEAN PATENT APPLICATION

(11) **EP 4 763 065 A1**
(43) Date of publication of application: **24.06.2026**
(21) Application number: 24221985.5
(22) Date of filing: 20.12.2024
(51) Int. Cl.: A61B 5/024, A61B 5/1455, A61B 5/00

(54) **WEARABLE MEDICAL DEVICE FOR RETRIEVING BIOLOGICAL SIGNALS**

(71) Applicant: Onera Technologies B.V., 5617 BD Eindhoven (NL)
(72) Inventor: Cramer, Peter, 5617BD Eindhoven (NL)
(74) Representative: AWA Sweden AB

(57) **Abstract**

The present invention relates to a wearable medical device (1) for retrieving biological signals, the device comprising a sensor (112), more particularly an optical sensor, such as a Photoplethysmography, PPG, sensor; an adhesive layer (15) attached to a contact surface (1121) of the sensor (112) and configured to be attached to a skin surface (2); a housing (110) attached to the adhesive layer (15), wherein the sensor is partially or fully enclosed by the housing (110) and the adhesive layer (15); and a spring (111) arranged between the housing (110) and the sensor (112). When the wearable medical device (1) is attached to a skin surface (2) by the adhesive layer (15), the spring (111) is configured to push the contact surface (1121) of the sensor (112) towards the skin surface (2).

## Description

### Technical Field

The present disclosure relates to a wearable medical device for retrieving biological signals.

### Background

Wearable medical devices are worn by individuals to monitor health and fitness metrics. These devices retrieve biological signals from the person wearing them and can retrieve, for example, heart rate, and blood oxygen levels, to name a few.

The result provided by these device can then be used by, for example, health care providers to diagnose patients and create treatment plans.

Optical wearable medical devices uses light-based technologies to track different types of health metrics with the help of optical sensors. Optical sensors can non-invasively retrieve biological signals.

One common type of optical sensors are photoplethysmography (PPG) sensors. PPG sensors monitor heart rate by using light to detect changes in blood volume within the microvascular bed of tissue.

When using wearable medical devices, it is important to ensure that the sensor accurately retrieves the biological signals. Factors such as contact between the sensor and the skin surface, and the presence of venous blood may affect the accuracy of the measurement result.

It is therefore a need to further improve wearable medical devices to ensure that accurate measurement results is provided.

### Summary

It is an object of the present invention to provide an improved solution that alleviates the mentioned drawbacks with present devices. Furthermore, it is an object to provide a wearable medical device that improve the accuracy and quality of a measurement.

The invention is defined by the appended independent claims, with embodiments being set forth in the appended dependent claims, in the following description and in the drawings.

According to an aspect of the invention, there is provided a wearable medical device for retrieving biological signals, the device comprising a sensor, more particularly an optical sensor, such as a Photoplethysmography, PPG, sensor; an adhesive layer attached to a contact surface of the sensor and configured to be attached to a skin surface; a housing attached to the adhesive layer, wherein the sensor is partially or fully enclosed by the housing and the adhesive layer; and a spring arranged between the housing and the sensor. When the wearable medical device is attached to a skin surface by the adhesive layer, the spring is configured to push the contact surface of the sensor towards the skin surface.

By "contact surface of the sensor" it may be meant a measurement interface surface, in particular an optical measurement interface surface, of the sensor. The measurement interface surface may be configured to be a skin surface of a user when the wearable medical device is arranged thereto.

By "the sensor is partially or fully enclosed by the housing and the adhesive layer" it may be meant that the sensor may be arranged between the housing and the adhesive layer such that at least two or more sides of the sensor is partially surrounded or surrounded by either the housing or the adhesive layer; or all sides of the sensor is partially surrounded or surrounded by either the housing or the adhesive layer.

The housing may be directly attached or indirectly attached to the adhesive layer.

Providing a wearable medical device comprising a spring configured to push the contact surface of the sensor towards a skin surface may enable improved accuracy of the measurement result of the sensor. Pushing the contact surface of the sensor towards the skin surface may limit the amount of venous blood that can negatively affect the measurement, in particular SpO2 measurement, and thereby improving the accuracy of the measurement result of the sensor. Pushing the contact surface of the sensor towards the skin surface may improve contact between the sensor and the skin surface and thereby improve the accuracy of the measurement result of the sensor.

The sensor may be rotationally movable relative to the housing. The sensor may be directly or indirectly attached to the housing. The sensor may be movably attached to the housing.

Enabling the sensor to be rotationally movable relative the housing may allow for the contact surface of the sensor to follow the contour of the skin surface, ensuring good skin contact and leading to improved accuracy of the measurement results of the sensor. It may mean that the sensor may be tilted relative to the housing, for instance if the skin surface at the location of the sensor does not extend completely in parallel with a normal extension of wearable medical device and/or the housing. The spring may be configured to enable different parts of the sensor to be pushed towards the skin surface by different amounts. The spring may have different contact points to the sensor, whereby different force may be applied to the sensor at the different contact points. This may conform the movement of the sensor to the shape of the skin surface.

The adhesive layer may cover the entire contact surface of the sensor. Providing the adhesive layer covering the entire contact surface may ensure that the sensor is securely attached to the skin surface and thereby improving the accuracy of the measurement result of the sensor. The adhesive layer being attached to both the skin surface and the entire contact surface of the sensor may improve the adaptability of the contact surface to irregularities and/or movements of the skin surface, thereby improving measurement quality.

The adhesive layer may comprise a sensor adhesive layer and/or a skin adhesive layer. The skin adhesive layer may ensure that the sensor is securely attached to the skin surface and may ensure good skin contact between the sensor and the skin surface, thereby improving the accuracy of the measurement result of the sensor.

The sensor adhesive layer may ensure that the sensor is securely attached to the skin surface and may ensure good skin contact between the sensor and the skin surface, thereby improving the accuracy of the measurement result of the sensor.

The wearable medical device comprising a skin adhesive layer may simplify the process of attaching the device to a skin surface since no external adhesive is needed.

The adhesive layer may comprise a conformable layer. By "conformable layer" it may be meant a layer comprising a gel or gel-like layer, or a flexible and/or stretchable film layer, which deforms and/or conforms depending on the position and/or rotation of the sensor, and/or depending on the skin surface, due to the added pressure from the spring. The conformable layer may ensure good contact between the sensor and the skin surface and thereby improving the accuracy of the measurement result of the sensor.

The adhesive layer and/or conformable layer may be substantially transparent. By "substantially transparent" it may be meant that the conformable layer let about 50% of emitted light to pass through, preferably about 60% of emitted light, about 70% of emitted light, about 80% of emitted light, about 90% of emitted light, about 93% of emitted light, about 95% of emitted light, about 97% of emitted light, or about 100% of emitted light.

Providing a substantially transparent conformable layer may minimize the absorption of emitted light by the adhesive layer or conformable layer and may thereby improve the accuracy of the measurement result of the sensor, especially in combination with the spring providing the position of the sensor to be adapted to the shape of the skin surface.

The conformable layer may cover the entire contact surface of the sensor. The conformable layer may be formed out of a polymer material, in particular polyurethane.

The conformable layer may be arranged between the sensor adhesive layer and the skin adhesive layer. The conformable layer may be laminated with the skin adhesive layer. The conformable layer may be laminated with the sensor adhesive layer.

The spring may be formed out of silicone, rubber or silicone rubber. The spring being formed out of silicone, rubber or silicone rubber may ensure that the spring has a high temperature resistance leading to prolonged storage under light pressure, while maintaining its spring characteristics.

The spring may have a substantially circular, oval, rectangular or square shape. The spring may have a first contact portion configured to be in contact with or attached to the housing. The spring may further have a second contact portion configured to be in contact with or attached to the sensor. The first and/or the second contact portions may have a plurality of contact points to the housing and/or the sensor. Alternatively, the first contact portion may be a continuous contact portion, for instance in a circular, oval, rectangular or square shape. Correspondingly, the second contact portion may be a continuous contact portion, for instance in a circular, oval, rectangular or square shape. The circumference of the first continuous contact portion may be larger than the second continuous contact portion. One of the first and second contact portions may be continuous while the other one is not. Between the first contact portion and the second contact portion the spring may substantially conical or at least partly conical. In one embodiment, the first contact portion may be a continuous contact portion, and the second contact portion may comprise two or more contact sub-portions formed as two or more contact points or contact areas. The two or more contact sub-portions may be formed with a distance to each other. The two or more contact sub-portions may be configured to be in contact with two or more remote portions of the sensor, for instance on a top surface of the sensor.

The housing may be rigid. The housing may be formed out of plastic, silicone, metal and/or rubber. The housing may be substantially circular shaped, substantially oval shaped, substantially rectangular shaped or substantially square shaped.

The wearable medical device may further comprise a liner configured to push the sensor in an opposite direction relative the direction the spring pushes the contact surface of the sensor. The liner may be configured to keep the spring in a compressed state prior to arrangement of the wearable medical device to a skin surface. The liner may be configured to be removed from the wearable medical device before attaching the wearable medical device to a skin surface. The liner may hold the sensor in place before use. The liner may protect the adhesive layer and the skin adhesive layer before use.

### Brief Description of the Drawing

The invention will in the following be described in more detail with reference to the enclosed drawings, wherein:
Fig. 1 shows a perspective view of a wearable medical device.
Fig. 2 device shows a side view of a wearable medical device.
Fig. 3 shows a front view of a wearable medical device.
Fig. 4 shows an exploded view of a wearable medical.
Fig. 5 shows a perspective view of a spring of a wearable medical device.
Figs. 6a-6b show cross-sectional side views of a wearable medical device.

### Description of Embodiments

The present invention will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. In the drawings, like numbers refer to like elements.

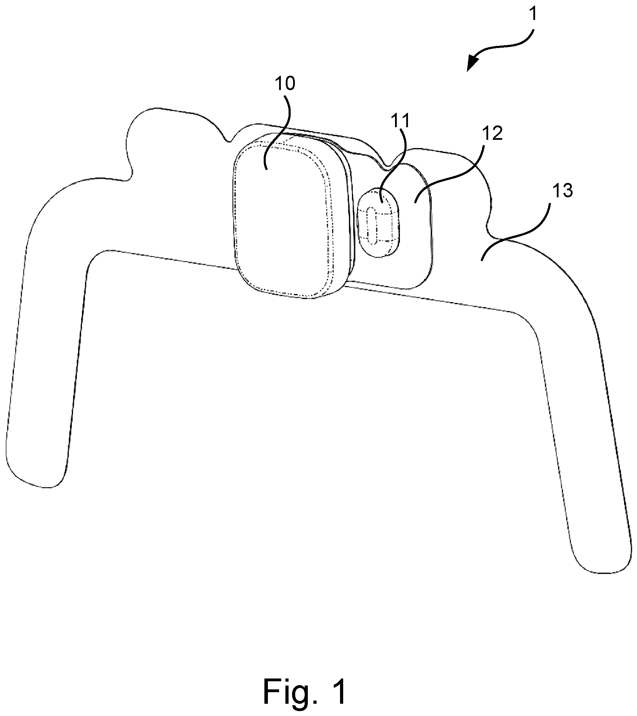

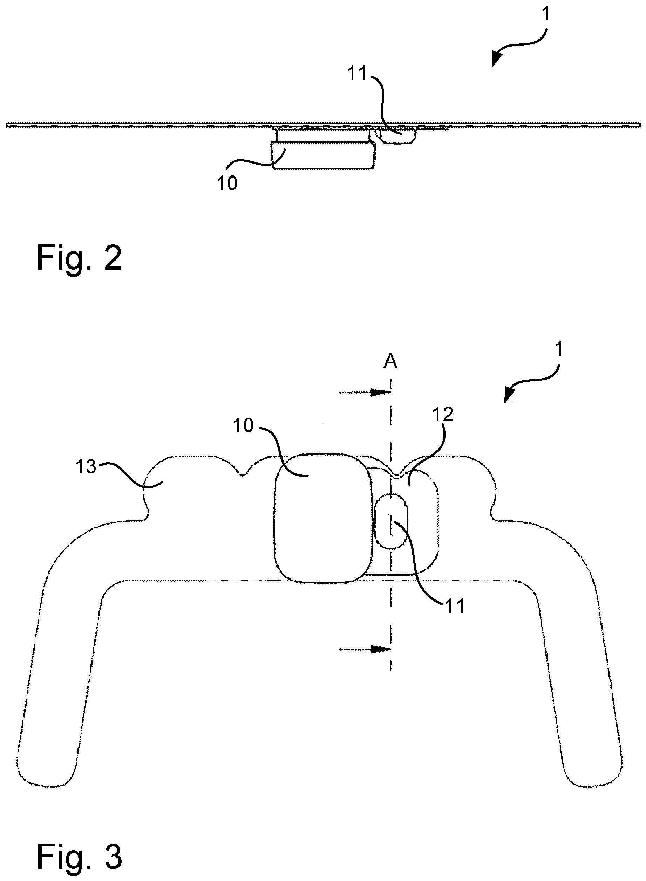

Figs. 1-3 illustrate different views of a wearable medical device 1. The wearable medical device 1 may be a patch. The wearable medical device 1 is configured to be attached to a skin surface. The wearable medical device shown in figs. 1-3 is configured to be attached to the skin surface of a forehead. However, the wearable medical device 1 may be configured to be attached to the skin surface of body parts such as the chest, the arm, the leg, and/or the stomach.

The wearable medical device 1 may be configured to retrieve biological signals. The wearable medical device 1 may be configured to retrieve optical signals, such as PPG signals. However, the wearable medical device 1 may not be limited to only retrieving optical signals. Alternatively, the wearable medical device 1 may be configured to retrieve, for example, thermal signals, such as temperature.

The wearable medical device 1 may comprise an electronics module 10. The electronics module 10 may comprise, for example, a battery. The battery may be rechargeable. The wearable medical device 1 may comprise a sensor module 11. The sensor module 11 may be a PPG module. The electronics module 10 may be connected to the sensor module 11. The electronics module 10 may be configured to provide the PPG module 11 with electricity.

The electronics module 10 and/or the sensor module 11 may be rectangular shaped, oval shaped, square shaped and/or circular shaped.

The wearable medical device 1 may comprise an adhesive strip 13. The adhesive strip 13 may be configured to be attached to a skin surface. The adhesive strip 13 may be formed out of rubber, silicone, foam, plastics, and/or fabric.

The wearable medical device 1 may comprise a module fastener 12. The module fastener 12 may attach the electronics module 10 and/or the sensor module 11 to the adhesive strip 13. The module fastener 12 may be configured to attach other modules to the adhesive strip 13.

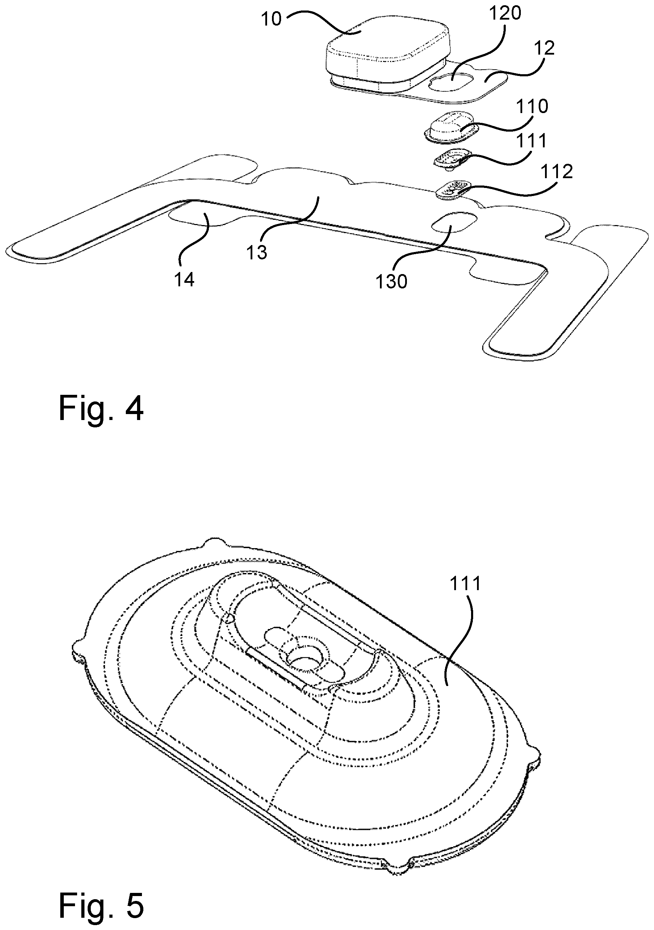

An exploded view of the wearable medical device is illustrated in fig. 4.

The wearable medical device 1 comprises a sensor 112. The sensor 112 may be an optical sensor, such as a PPG sensor. Alternatively, the sensor 112 may be a temperature sensor. The wearable medical device 1 comprises a housing 110. The wearable medical device 1 comprises a spring 111. The spring 111 is arranged between the housing 110 and the sensor 112.

The sensor module 11 may comprise the sensor 112, the housing 110, and the spring 111.

The housing 110 may be rigid. The housing 110 may be formed out of plastic, silicone, metal and/or rubber. The housing 110 may be rectangular shaped, square shaped, oval shaped, or circular shaped. The housing 110 may be shaped as a half sphere, a half ellipsoid, a cube, or a rectangular cuboid. The housing 110 may be formed out of one piece or out of multiple pieces. The multiple pieces may be formed out of the same material or different materials.

The wearable medical device 1 is illustrated in fig. 4 as comprising a liner 14. The liner 14 may be removably attached to the adhesive strip 13. The liner 14 may be configured to be removed from the wearable medical device 1 before attaching the wearable medical device 1 to a skin surface. The liner 14 illustrated in fig. 4 have a surface which is larger than a surface of the adhesive strip 13. However, the surface of the liner 14 may be the same size and/or have the same shape as the adhesive strip 13. The liner 14 may be formed out of plastic.

The adhesive strip 13 may comprise a hole 130. The hole 130 may be rectangular shaped, oval shaped, square shaped, or circular shaped. The hole 130 may have the same shape as the sensor module 11. The hole 130 may have the same shape as the sensor 112. The sensor 112 may be arranged inside the hole 130 of the adhesive strip 13.

The module fastener 12 may comprise a hole 120. The hole 120 of the module fastener 12 may be rectangular shaped, oval shaped, square shaped, or circular shaped. The hole 120 of the module fastener 12 may have the same shape as the sensor module 11. The hole 120 of the module fastener 12 may have the same shape as the housing 110.

Fig. 5 Illustrates the spring 111 of the wearable medical device 1. The spring 111 may be formed out of silicone, rubber and/or silicone rubber. Alternatively, the spring 111 may be a mechanical spring or a foam spring. The mechanical spring may be formed out of metal.

The spring 111 is illustrated in fig. 5 as being oval shaped. However, the spring 111 may be rectangular shaped, square shaped or circular shaped.

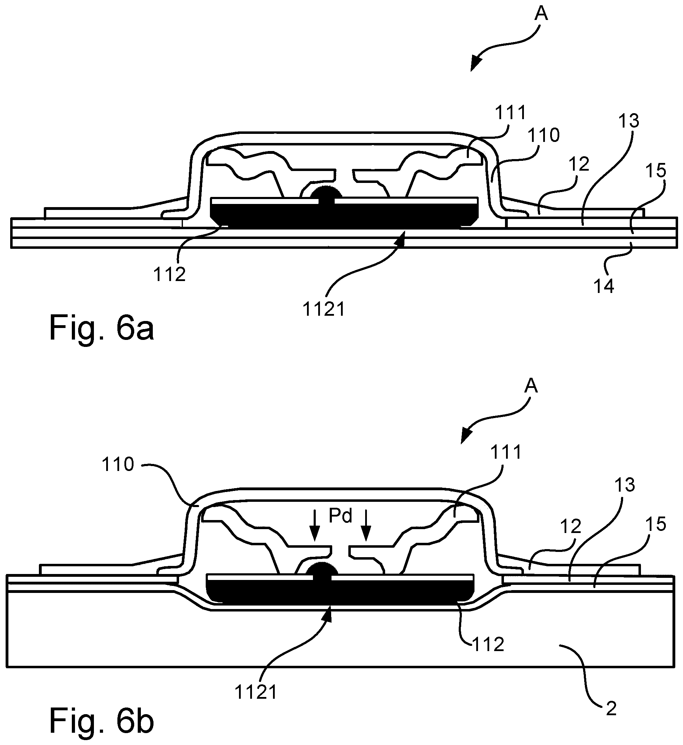

Fig. 6a-6b illustrate cross-section A of the wearable medical device 1.

The wearable medical device 1 comprises an adhesive layer 15. The sensor 112 comprises a contact surface 1121. The adhesive layer 15 is attached to the contact surface 1121 of the sensor 112. The adhesive layer 15 may cover the entire contact surface 1121 of the sensor 112. The adhesive layer 15 may be attached to the adhesive strip 13. The adhesive layer 15 may cover the entire surface of the adhesive strip 13. The adhesive layer 15 may partially cover the surface of the adhesive strip 13.

The adhesive layer 15 is configured to be attached to a skin surface 2.

The housing 110 is attached to the adhesive layer 15. In figs. 6a-6b, the housing 110 is shown as being indirectly attached to the adhesive layer 15, i.e. the module fastener 12 attaches the housing 110 to the adhesive strip 13 which is attached to the adhesive layer 15. However, the housing 110 may be indirectly attached to the adhesive layer 15 in other ways, for example, by the housing 110 being attached to the module fastener 12 which is attached to the adhesive layer 15, or by the housing 110 being attached to the adhesive strip 13 which is attached to the adhesive layer 15.

Alternatively, the housing 110 may be directly attached to the adhesive layer 15.

The sensor 112 is partially or fully enclosed by the housing 110 and the adhesive layer 15. The sensor 112 may be movably attached to the housing 110. The sensor 112 may be directly or indirectly attached to the housing 110. The sensor 112 may be rotationally movable relative the housing 110.

The spring 111 may be attached to the housing 110. The spring 111 may be attached to the sensor 112. The sensor 112 may be attached to the housing 110 by the spring 111.

Fig. 6b illustrates the spring 111 pushing the contact surface 1121 towards the skin surface 2. When the wearable medical device 1 is attached to the skin surface 2 by the adhesive layer 15, the spring 111 is configured to push the contact surface 1121 of the sensor 112 towards the skin surface 2.

The liner 14 may be configured to push the sensor 112 in an opposite direction relative a direction Pd the spring 111 pushes the contact surface 1121 of the sensor 112.

The adhesive layer 15 may comprise a sensor adhesive layer and/or a skin adhesive layer. The sensor adhesive layer may be arranged in the hole 130 of the adhesive strip 13. The sensor adhesive layer may only be arranged in the hole 130 of the adhesive strip 13. The sensor adhesive layer may be attached to the contact surface 1121 of the sensor 112.

The adhesive layer 15 may comprise a conformable layer. The conformable layer may be substantially transparent. The adhesive layer 15 may be substantially transparent. The conformable layer may cover the entire contact surface 1121 of the sensor 112. The conformable layer may be formed out of a polymer material, in particular polyurethane. The conformable layer may be arranged between the sensor adhesive layer and the skin adhesive layer. The conformable layer may be arranged in the hole 130 of the adhesive strip 13. The conformable layer may cover the entire surface of the adhesive strip 13. The conformable layer may only be arranged in the hole 130 of the adhesive strip 13.

The spring 111 may have a first contact portion configured to be in contact with or attached to the housing. The spring 111 may further have a second contact portion configured to be in contact with or attached to the sensor 112. The first and/or the second contact portions may have a plurality of contact points to the housing 110 and/or the sensor 112. Alternatively, the first contact portion may be a continuous contact portion, for instance in a circular, oval, rectangular or square shape. Correspondingly, the second contact portion may be a continuous contact portion, for instance in a circular, oval, rectangular or square shape. The circumference of the first continuous contact portion may be larger than the second continuous contact portion. One of the first and second contact portions may be continuous while the other one is not. Between the first contact portion and the second contact portion the spring may substantially conical or at least partly conical. The shape and size of the at least partly conical extension of the spring 111 between the two contact portions may configured such that a desired spring behavior of the spring 111 is achieved. The first contact portion may face in a direction substantially perpendicular to a direction in which the second contact portion may face.

As seen in fig. 5, the first contact portion may be a continuous contact portion, and the second contact portion may comprise two contact sub-portions formed as two contact points or contact areas. The two contact sub-portions may be separated and formed with a distance to each other. The two contact sub-portions may be configured to be in contact with two remote portions of the sensor 112, on a top surface of the sensor 112 as seen in figs. 6a-b. The first contact portion may be a continuous contact portion extending in a shape and size corresponding to an inner shape and size of the housing 110 to which the first contact portion may be attached, as seen in figs. 6a-b.

In the drawings and specification, there have been disclosed preferred embodiments and examples of the invention and, although specific terms are employed, they are used in a generic and descriptive sense only and not for the purpose of limitation, the scope of the invention being set forth in the following claims.

## Claims

1. A wearable medical device (1) for retrieving biological signals, the device (1) comprises:
a sensor (112), more particularly an optical sensor, such as a Photoplethysmography, PPG, sensor,
an adhesive layer (15) attached to a contact surface (1121) of the sensor (112) and configured to be attached to a skin surface (2),
a housing (110) attached to the adhesive layer (15), wherein the sensor (112) is partially or fully enclosed by the housing (110) and the adhesive layer (15); and
a spring (111) arranged between the housing (110) and the sensor (112),
wherein, when the wearable medical device (1) is attached to a skin surface (2) by the adhesive layer (15), the spring (111) is configured to push the contact surface (1121) of the sensor (112) towards the skin surface (2).

2. The wearable medical device (1) according to claim 1, wherein the sensor (112) is rotationally movable relative the housing (110).

3. The wearable medical device (1) according to claim 1 or 2, wherein the adhesive layer (15) covers the entire contact surface (1121) of the of the sensor (112).

4. The wearable medical device (1) according to any one of the preceding claims, wherein the adhesive layer (15) comprises a sensor adhesive layer and/or a skin adhesive layer.

5. The wearable medical device (1) according to any one of the preceding claims, wherein the adhesive layer (15) comprises a conformable layer.

6. The wearable medical device (1) according to claim 5, wherein the conformable layer is substantially transparent.

7. The wearable medical device (1) according to claim 5 or 6, wherein the conformable layer covers the entire contact surface (1121) of the sensor (112).

8. The wearable medical device (1) according to any one of claims 5 to 7, wherein the conformable layer is formed out of a polymer material, in particular polyurethane.

9. The wearable medical device (1) according to any one of claims 5 to 8 when dependent on claim 4, wherein the conformable layer is arranged between the sensor adhesive layer and the skin adhesive layer.

10. The wearable medical device (1) according to any one of claims 5-9, wherein the conformable layer comprises a flexible and/or stretchable film configured to deform and/or conform depending on a skin surface (2) following added pressure towards the skin surface by the spring (111)

11. The wearable medical device (1) according to any of the preceding claims, wherein the spring (111) is formed out of silicone, rubber or silicone rubber.

12. The wearable medical device (1) according to any of the preceding claims, wherein the housing (110) is rigid.

13. The wearable medical device (1) according to any one of the preceding claims, wherein the housing (110) is formed out of plastic, silicone, metal and/or rubber.

14. The wearable medical device (1) according to any one of the preceding claims, wherein the housing (110) is substantially circular shaped, substantially oval shaped, substantially rectangular shaped or substantially square shaped.

15. The wearable medical device (1) according to any one of the preceding claims, further comprising a liner (14) configured to push the sensor (112) in an opposite direction relative the direction (Pd) the spring pushes the contact surface (1121) of the sensor (112), and
wherein the liner (14) is configured to be removed from the wearable medical device (1) before attaching the wearable medical device (1) to a skin surface (2).
